(19)

Europäisches
Patentamt
European
Patent Office
Office européen
des brevets

(11) **EP 4 063 520 A1**

(12) **EUROPEAN PATENT APPLICATION**
published in accordance with Art. 153(4) EPC

(43) Date of publication:
**28.09.2022 Bulletin 2022/39**

(21) Application number: **20889629.0**

(22) Date of filing: **15.09.2020**

(51) International Patent Classification (IPC):
***C12Q 1/6851*** (2018.01)

(86) International application number:
**PCT/KR2020/012411**

(87) International publication number:
**WO 2021/101049 (27.05.2021 Gazette 2021/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.11.2019 KR 20190149391**

(71) Applicants:
• **U-GENE&CELL CO.**
**Seoul 05557 (KR)**
• **Boditech Med Inc.**
**Chuncheon-si, Gangwon-do 24398 (KR)**

(72) Inventors:
• **WON, Byoung Yeon**
**Seoul 06544 (KR)**

• **KIM, Su Sung**
**Incheon 21970 (KR)**
• **AN, Jae Un**
**Incheon 22003 (KR)**
• **IM, Youn Tae**
**Chuncheon-si, Gangwon-do 24401 (KR)**
• **PARK, Sang Hyun**
**Seoul 07360 (KR)**
• **PARK, Jae Ho**
**Seoul 06335 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **METHOD FOR DETECTING AND QUANTIFYING TARGET NUCLEIC ACID IN REAL TIME USING SINGLE SIGNAL FLUORESCENT MATERIAL**

(57)     The present disclosure relates to a method for detecting and quantifying a nucleic acid in real time and at high speed. The present disclosure can provide a real-time high-speed PCR method in which fluorescent signals can be measured from a single-wavelength light source by using a single signal fluorescent material under continuous temperature control. Thus, the PCR method can be performed with a compact lightweight device with a simplified structure.

[fig.6]

EP 4 063 520 A1

```
<110>      U-GENE&CELL Co.
           Boditech Med Inc.

<120>      Methods for real-time detecting and quantifying of target nucleic
           acid using single signal fluorescent material

<130>      X20E10C0155

<150>      KR 10-2019-0149391
<151>      2019-11-20

<160>      2

<170>      KoPatentIn 3.0

<210>      1
<211>      22
<212>      DNA
<213>      Artificial Sequence

<220>
<223>      primer_F


<400>      1
gtctgcggaa ccggtgagta ca                                                    22


<210>      2
<211>      23
<212>      DNA
<213>      Artificial Sequence

<220>
<223>      primer_R


<220>
<221>      misc_feature
<222>      (4)
<223>      R represents A or G.


<220>
<221>      misc_feature
<222>      (12)
<223>      R represents A or G.


<400>      2
cgcracccaa crctactcgg cta                                                   23
```

## Description

## Technical Field

The present disclosure relates to a method for detecting and quantifying a target nucleic acid at high speed in real time using a single signal fluorescent material.

## Background Art

Polymerase chain reaction (PCR) is a technology that can selectively amplify a specific nucleic acid in a biological sample and is composed of DNA denaturation, annealing, and DNA extension steps. In such a PCR process, a reaction product containing a sample usually requires repeated heating and cooling several tens of times. In particular, one of the important factors determining the overall reaction time in a PCR reaction is the time it takes for the reactants to be heated to a predetermined temperature or cooled to a predetermined temperature. Conventionally, the PCR reaction is maintained at a specific temperature for a predetermined time to perform each step of the PCR reaction for heating and cooling, and a temperature change interval according to the transition from one step to the next is not considered in the reaction. In this case, there is a disadvantage in that the time required for the total PCR reaction increases because time such as a temperature change interval is required.

In addition, in order to detect a specific nucleic acid by PCR reaction, the target sequence must be amplified, and at the same time, the internal control sequence must be amplified. This is required as a step of confirming whether the target sequence is not included in the actual sample when the target sequence is not detected or whether the amplification reaction is not performed properly due to a problem in the PCR reaction.

On the other hand, in the case of a real time PCR device, there is a disadvantage in that only a fluorescence signal of a single wavelength may be measured in order to reduce unit cost, miniaturize, and reduce measurement time. As described above, when only the fluorescence signal of a single wavelength can be measured, the fluorescence signal of the amplification product for the target sequence and the fluorescence signal of the amplification product for the internal control sequence cannot be simultaneously measured. In general, the target sequence and the internal control sequence can be distinguished using probe materials modified with fluorescent materials of different wavelengths, which cannot be distinguished if only fluorescent signals of a single wavelength can be measured.

In order to overcome this disadvantage, there is a need to the development of a method capable of distinguishing the amplification of a target sequence from the amplification of an internal control sequence with a single wavelength fluorescence measurement module. KR Patent Publication No. 10-2016-0126092 discloses a technique for detecting a plurality of target sequences using only a single type of label in one reaction vessel using different detection temperatures. However, although the literature uses a single type of labeling substance, two types of probes are being used to detect two nucleic acids with different detection temperatures, so two types of labeling substances are actually being used. Therefore, a technology for distinguishing a target sequence from an internal control group sequence using only a single signal fluorescent material is not disclosed.

## Disclosure

## Technical Problem

The objective of the present disclosure is to provide a method for amplifying and quantifying nucleic acids in real time and at high speed.

## Technical Solution

The inventors of the present application confirmed that it is possible to amplify and quantify nucleic acids in real time and at high speed by using a single signal fluorescent material and an internal control sequence together in a PCR device of a kinetic paradigm while researching a high-speed PCR method.

As a result of the study, the present disclosure has been completed by not only confirming whether false negative is generated through an internal control group sequence under continuous temperature control but also confirming that multiple targets can be detected at a single wavelength in real time and at high speed using a single signal fluorescent material, and amplification curve measurement and melting curve measurement can be performed.

Accordingly, the present disclosure relates to a method for detecting and quantifying a target nucleic acid in real time and at high speed by measuring fluorescence at a single wavelength.

Hereinafter, the configuration of the present disclosure will be specifically described.

The present disclosure provides a method for detecting and quantifying a target nucleic acid in real time, the method including:

> (a) repeatedly performing DNA denaturation, annealing,
> and DNA extension of a target sequence and an internal control sequence using a PCR reaction solution containing a single-signal fluorescent material and a primer pair that specifically bind to the target sequence under continuous temperature control; and
> (b) measuring a fluorescence signal at regular intervals from the start of heating for the DNA denaturation to the completion of the DNA denaturation.

In the present disclosure, the single signal fluorescent material in step (a) may be an intercalating dye. The intercalating dye does not bind to single-stranded DNA (ssDNA) but emits fluorescence when it binds to double-stranded DNA (dsDNA) formed by DNA extension after primer binding (annealing). Therefore, the intercalating dye can quantify the amplification product by measuring the fluorescence signal in the annealing or DNA extension step.

The intercalating dye can be used to quantify the amplification product of an internal control sequence due to the non-specific property of binding to all nucleic acid sequences or to determine whether the PCR reaction is false negative by using the melting temperature (Tm).

The intercalating dye may be SYBR Green I, SYBR Gold, YoYo, Evagreen, or LCGreen but is not limited thereto.

In one embodiment, the dye may be a SYBR-based intercalating dye.

In the present disclosure, the primer pair that specifically binds to the target sequence in step (a) may be one represented by the nucleic acid sequences of SEQ ID NO: 1 and 2:

> [SEQ ID NO: 1] 5'- GTCTGCGGAACCGGTGAGTACA -3'
> [SEQ ID NO: 2] 5'- CGCRACCCAACRCTACTCGGCTA -3'
> In the SEQ ID NO: 2, R is A or G.

In the present disclosure, the primer is used for initiation of a PCR reaction and refers to an oligonucleotide or polynucleotide that hybridizes complementary to a template DNA. The primer for PCR reaction may be a pair of forward primers (or sense primers) selected from the same sense strand as the gene code progression direction of the nucleic acid molecule being amplified and reverse primers (or antisense primers) selected from antisense strands complementary to the sense strand.

In the present disclosure, the internal control sequence amplified together with the target sequence may be designed to have a lower melting temperature (Tm) than the amplification product of the target sequence. In the present disclosure, the internal control sequence may be included for the purpose of eliminating false negatives in the PCR reaction. For example, if the target sequence is not detected after the PCR reaction, it is essential to check whether the target sequence is not included in the sample or whether the PCR reaction is not performed properly. Accordingly, the internal control sequence is a sequence to be always detected, and if the internal control sequence is not detected after the PCR reaction, it should be determined that the PCR reaction has not been performed properly.

In the present disclosure, the melting refers to the separation of double-stranded DNA (dsDNA) into single-stranded DNA (ssDNA) as the temperature increases, and accordingly, the intensity of fluorescence changes with time. A graph representing this relationship is called a melting curve. The melting temperature (Tm) refers to a midpoint in the melting curve and a temperature at which 50% of double-stranded DNA (dsDNA) is separated into single-stranded DNA (ssDNA) . That is, the melting temperature (Tm) is a temperature at which the fluorescence signal rapidly decreases, and the melting temperature is a temperature at which fluorescence emitted from the intercalating dye is hardly detected.

In the present disclosure, the PCR reaction solution may include DNA polymerase (DNTP), a deoxynucleotide mixture (dNTP), a buffer solution, or magnesium chloride ($MgCl_2$) required for the PCR reaction, as well as a primer pair specifically bonded to the single signal fluorescent material and a target sequence.

In the present disclosure, the continuous temperature control of step (a) may be performed in a device capable of performing a PCR reaction of a kinetic paradigm. The device may be a real time PCR device. The real time PCR device refers to a device capable of amplifying a nucleic acid having a specific nucleic acid sequence and simultaneously measuring and analyzing the presence or absence and extent of a PCR amplification product in real time.

In general, PCR reaction can be divided into equilibrium and kinetic paradigms according to the state of temperature. In the case of the PCR reaction of the equilibrium paradigm, as a method mainly used in the related art, the steps of DNA denaturation, annealing, and DNA extension for nucleic acid amplification are maintained at a specific temperature for a certain time, and the temperature change interval according to the transition from one step to the next step is not taken into account in the response. On the other hand, in the PCR reaction of a dynamic paradigm such as the present disclosure, each step for nucleic acid amplification occurs over a specific temperature. That is, in the case of the kinetic paradigm, temperature control is performed continuously. Accordingly, the reaction rate of each step, such as DNA denaturation, annealing, and DNA extension, is only dependent on temperature, and one or more reactions, such as annealing and DNA extension, may occur simultaneously in the temperature change interval.

Accordingly, the method, according to this disclosure, uses a temperature control method in a nucleic acid amplification reaction in which the DNA denaturation step is performed at a first temperature; and the annealing and/or DNA extension step is performed at a second temperature are repeated several times.

In one embodiment, the continuous temperature control may be performed by including:

heating the reaction vessel to a first temperature by contacting the reaction vessel containing the PCR reaction solution with a heating block; and

cooling the reaction vessel to a second temperature by exposing the separated reaction vessel to artificial air flow for a predetermined period of time after separating the heated reaction vessel from the heating block.

In this case, the first temperature refers to a temperature at which DNA denaturation is performed, and the second temperature refers to a temperature at which annealing and/or DNA extension is performed. That is, at the second temperature, only annealing may be performed, or annealing and DNA extension may be performed simultaneously. In this case, the steps of heating the reaction vessel to the first temperature and cooling the reaction vessel to the second temperature may be repeated several times. At this time, when only annealing is performed at the second temperature, the step of cooling the reaction vessel and then allowing the reaction vessel to stand at the fourth temperature for a predetermined time for DNA extension may be further included.

In another embodiment, the continuous temperature control may be performed by including:

heating the reaction vessel at a first temperature by contacting the reaction vessel containing the PCR reaction solution with a heating block that is maintained constantly at a first temperature;

cooling the reaction vessel to a third temperature by exposing the separated reaction vessel to artificial air flow for a predetermined period of time after separating the heated reaction vessel from the heating block; and

separating the cooled reaction vessel from the heating block after the cooled reaction vessel is brought into contact with the heating block and heated to a second temperature.

In this case, the first temperature refers to a temperature at which DNA denaturation is performed, the third temperature refers to a temperature at which annealing is performed, and the second temperature refers to a temperature at which DNA extension is performed.

In the present disclosure, the steps of DNA denaturation, annealing, and DNA extension are shown as one cycle.

In the method according to the present disclosure, in the step of heating the reaction vessel to the first temperature, the reaction vessel containing the PCR reaction solution may be brought into contact with a heating block maintained at a specific temperature for a predetermined time to be heated for a predetermined time to reach the desired denaturation temperature. In this case, the heating block may maintain a temperature of 90°C or higher. In the step of cooling the reaction vessel to the second temperature, either the reaction vessel or the heating block may be moved in a specific direction to separate the reaction vessel from the heating block, and then the reaction vessel may be in contact with the artificial air flow generated from the blowing fan for a predetermined time to cool the reaction vessel so that the separated reaction vessel may reach a desired cooling temperature. At this time, by adjusting the contact time with the heating block or the artificial air flow generated from the blowing fan, the temperature of the reaction solution in the reaction vessel can be controlled to reach the desired temperature. By repeating this process, the temperature of the reaction solution may be adjusted to repeat a temperature section within a specific range.

In one embodiment, the artificial air flow may be wind having room temperature. Although the temperature of the room temperature may be different for each environment, the speed of the wind may be controlled by adjusting the rpm of a fan generating wind by attaching a thermometer for measuring an outdoor temperature to the device. In addition, when used indoors, considering the temperature condition of PCR, there was little difference in the time for cooling the reaction vessel at a temperature of 20°C, 30°C wind, or 35°C wind, which is a temperature above room temperature. However, wind speed may affect cooling time.

In the present disclosure, the time required for cooling the heated reaction solution for DNA denaturation to annealing and/or DNA extension temperature, that is, the predetermined time the reaction vessel is exposed to artificial air flow, is related to the speed of the wind sprayed toward the reaction vessel, and the cooling time can be determined by the following general Formula:

$$t = 4 + 2 * e^{-(v-7.4)/6.2}$$

In the above general Formula, t is the time required for cooling, and v means the speed of the wind.

In the present disclosure, the temperature is based on the reaction solution or reactant contained in the reaction vessel. However, due to the characteristics of a PCR reaction, considering a general PCR tube having a very small volume of about 1 to 50 $\mu$l and excellent heat transfer, it may be considered that there is no difference between a temperature of a reaction vessel and a temperature of a reaction solution. Accordingly, unless otherwise specified, the temperature of the reaction vessel may be understood as the temperature of the reaction solution.

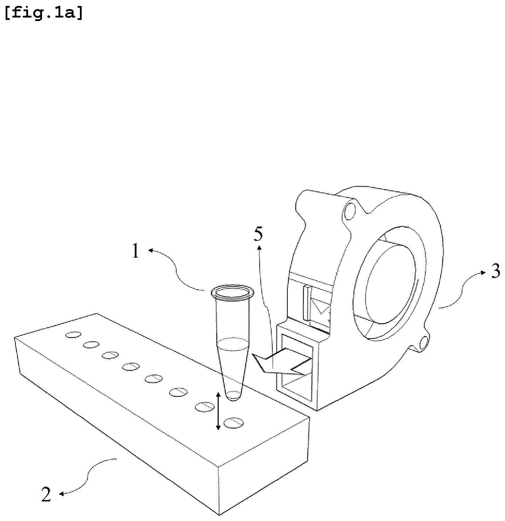

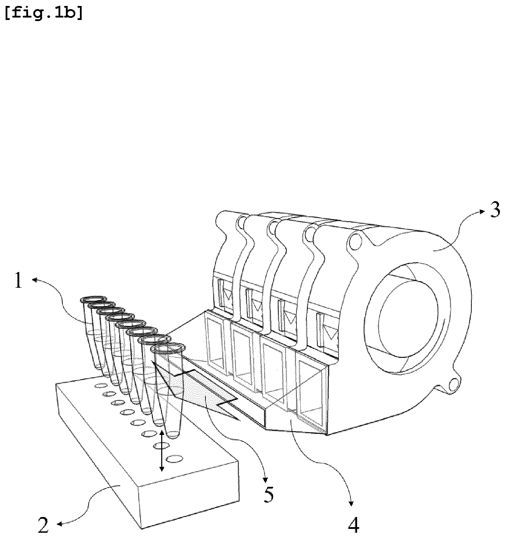

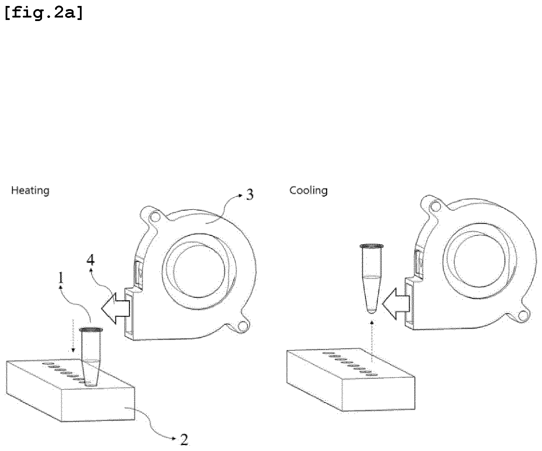

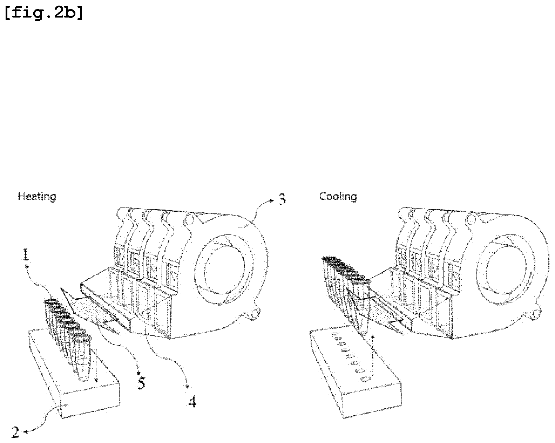

In one embodiment, the method according to the present disclosure can be performed using a device such as that shown in FIGS. 1 and 2. Specifically, a heating block 2 of metal material with holes formed in the shape of the reaction vessel so that the bottom of the reaction vessel 1 can be in complete contact with the heating block in which heating is maintained at a high temperature and a device with a blower fan 3 located above the heating block designed to provide wind (artificial airflow) 4 may be used. In the stage where the reaction vessel 1 containing the reaction solution is heated for DNA denaturation, the reaction solution is heated by contact with the heating block 2 and then moves upward and can be cooled by contact with the wind 4 generated from the blowing fan 3, respectively. In this case, heating and cooling of the reaction solution can be achieved with a very simple operation. That is, during heating, the reaction vessel is mounted on the heating block to perform rapid heating, and at the same time, the opening/closing block blocks the blowing nozzle to block cooling by the cooling wind or, if necessary, the wind may be continuously generated. In addition, during cooling, the heating block is separated from the reaction vessel to stop heating, and at the same time, the opening/closing block is separated from the blowing nozzle to open the blowing nozzle so that rapid cooling can be achieved by the cooling wind.

In the present disclosure, the reaction vessel may be changed depending on the configuration of the device in which the method according to the present disclosure is implemented, for example, a tube of 100 $\mu$l or 200 $\mu$l, a capillary tube, a microfluidic channel or a thin film chamber may be used. However, the present disclosure is not limited thereto.

In the present disclosure, the first temperature means a temperature required to separate double-stranded DNA into single-stranded DNA in a nucleic acid amplification reaction, and the first temperature may be determined by various factors of a composition of bases constituting DNA, such as the G/C composition ratio, total length, the concentration of salt contained in the buffer, and the like, and may be selected as an appropriate temperature by those skilled in the art. For example, the first temperature may be 90°C or higher, or 90 to 100°C, but is not limited thereto.

The heating block used to heat the reaction vessel to the first temperature may be to maintain the first temperature. That is since the heating block does not require a temperature change, the reaction rate can be increased. The heating block may be selected using a method known in the art, for example, a thermoelectric element (Peltier), a resistive heater, a heating coil, wind at a specific temperature, or liquid with high or low temperature or the like may be used, but is not limited thereto.

According to the method of the present disclosure, under the same PCR reaction conditions (tube and volume) as a conventional PCR method (e.g., a PCR reaction of an equilibrium paradigm), the method of the present disclosure may perform a PCR reaction faster than a conventional PCR method (e.g., a PCR reaction of an equilibrium paradigm). In addition, the method of the present disclosure can be applied to different PCR reaction conditions (tube and volume), and the method of the present disclosure can quickly perform a PCR reaction compared to the conventional PCR method (e.g., a Peltier-based temperature control method with one heating/cooling source).

In the PCR reaction conditions, when a different vessel and a different volume are applied, the ramping rate (degree of temperature change per second) may vary in the method according to the present disclosure, the heating/cooling time may be determined by obtaining a heating rate according to a volume change or a material property of the reaction vessel through an experiment.

In the present disclosure, the step of heating the reaction vessel to the first temperature includes contacting the reaction vessel and the heating block for a predetermined time, at this time, the predetermined time means a time when the temperature of the reaction solution contained in the reaction vessel contacts the heating block and reaches a temperature sufficient for the denaturation of the double-stranded DNA of the reaction solution. In this case, the predetermined time may be determined according to the volume of the reaction solution and may be determined as an appropriate time by those skilled in the art.

After the temperature of the reaction vessel reaches the first temperature, the reaction vessel and the heating block are separated from each other. During this process, the heating block or reaction vessel may be moved.

In one embodiment, the heating block may descend, or the reaction vessel may rise while the heating block is fixed.

The interval between the heating block and the separated reaction vessel minimizes the total reaction time depending on the time required for movement and is not limited as long as it is an appropriate interval for air provided from the excursion fan to contact the reaction vessel according to the position of the blower. For example, the interval may be an interval that does not affect the temperature maintenance of the heating block in contact, that is, the interval may be an interval in which the reaction vessel is separated from the air around the heated heating block by the heating block, and wind provided for cooling reaches directly and contacts the heating block, thereby not affecting temperature maintenance of the heating block.

In one embodiment, the interval may be 0.5 to 2 cm.

In the present disclosure, the separated reaction vessel may be exposed to an artificial air flow for a predetermined time for cooling. At this time, the artificial air flow is air at room temperature and may be provided through a fan-type device having a motor, for example, a general cooling fan, a blower fan, a cross fan, or an air compressor but is not limited thereto.

In the present disclosure, cooling the reaction vessel may be performed by moving the reaction vessel in an upward direction of the heating block up to a predetermined position in order to fix the position of the heating block and separate the reaction vessel from the heating block.

As described above, in the method of the present disclosure, annealing and DNA extension may be performed simultaneously in one step, or DNA denaturation, annealing, and DNA extension reactions may be performed individually. When DNA denaturation, annealing, and DNA extension are performed individually, the step of allowing the cooled reaction vessel to stand at room temperature for a certain period of time after cooling the reaction vessel may be further included.

As described above, the present disclosure can detect and quantify a target nucleic acid by performing step (b) of measuring a fluorescence signal at a specific time while performing a PCR reaction under continuous temperature control. More specifically, in step (b), the fluorescence signal is measured at regular time intervals from the start of heating to the time when DNA denaturation is completed, and the fluorescence signal measured at the Tx time point is selected from the measured fluorescence signal values. The fluorescence signal measured at the time point Tx refers to a fluorescence signal measured at a time point at which a temperature higher than the melting temperature of the amplification product for the internal control sequence is reached. At this time, the fluorescence signal (signal B) measured at the heating start time includes the fluorescence signal of the amplification product for the target sequence and the fluorescence signal of the amplification product for the internal control sequence, and the fluorescence signal (signal A) measured at the Tx time point may include only the fluorescence signal of the amplification product for the target sequence. In step (b), the predetermined time interval for measuring the fluorescence signal may be 0.5 to 1 second but is not limited thereto.

The Tx time point may vary depending on the melting temperature (Tm) of the internal control sequence. That is, since the Tx time point may be determined according to the target sequence to be detected, it may be applied universally. This is due to the temperature control method through time control presented in the present disclosure, that is, in the present disclosure, since the signal measurement temperature may be adjusted by adjusting the measurement time, even if the Tm value of the PCR product is changed due to a change in the target sequence, the target nucleic acid may be detected and quantified as long as only the Tx time point can be determined. In the case of a conventional probe, such as a TaqMan probe, whenever the target sequence is changed, it is inconvenient to apply a probe suitable for the target sequence every time.

In addition, the present disclosure may further include confirming whether a false negative occurs by checking whether the internal control sequence is amplified.

The method, according to the present disclosure, can analyze the amplification cuff and the melting curve at the same time through one reaction vessel and one PCR reaction. After PCR amplification, the temperature is gradually increased after the last DNA denaturation step, and the amplification of the target sequence and the internal control sequence may be confirmed by analyzing a melting curve indicating a decreasing fluorescence intensity of the fluorescent material as the double-stranded DNA is separated into single-stranded DNA according to thermal denaturation of the amplification product. That is, through the melting curve, one or more melting temperatures at which 50% of double-stranded DNA is separated into single-stranded DNA can be identified. Since the melting temperature varies depending on the length or arrangement of the nucleic acid sequence, the difference in melting temperature can distinguish the amplification product for the target sequence from the amplification product for the internal control sequence. In other words, the amplification products of multiple target sequences can be distinguished using the difference in the melting temperature.

Advantages and features of the present disclosure and methods for achieving them will become apparent with reference to the following embodiments described below in detail. However, the present disclosure is not limited to the embodiments disclosed below but will be implemented in a variety of different forms, and the present embodiments are provided to complete the disclosure of the present disclosure and to completely inform the scope of the present disclosure to those skilled in the art, and the present disclosure is only defined by the scope of the claims.

**Advantageous Effects**

The present disclosure may detect and quantify nucleic acid in real time and at high speed by measuring a fluorescent signal from a single-wavelength light source using a single-signal fluorescent material under continuous temperature control, thereby providing a real time high-speed PCR method with a simple structure, miniaturization, and light weight.

## Description of Drawings

FIGS 1A and 1B schematically show the appearance of a PCR device to which the method according to the present disclosure is applied, and the PCR device is composed of a reaction vessel 1, a heating block 2, and a blowing fan 3, and the reaction vessel 1 contained reaction solution is moved so as to be in contact with the heating block 2 and the wind 5 generated from the blowing fan, respectively;

FIGS. 2A and 2B schematically show a method in which heating and cooling are achieved in a PCR device to which the method according to the present disclosure is applied, the reaction vessel 1 is moved to the heating block 2 and brought into contact with the reaction solution is heated, and then the reaction vessel 1 move in front of the blowing fan 3 and comes into contact with the wind 5 generated from the blowing fan to cool the reaction solution;

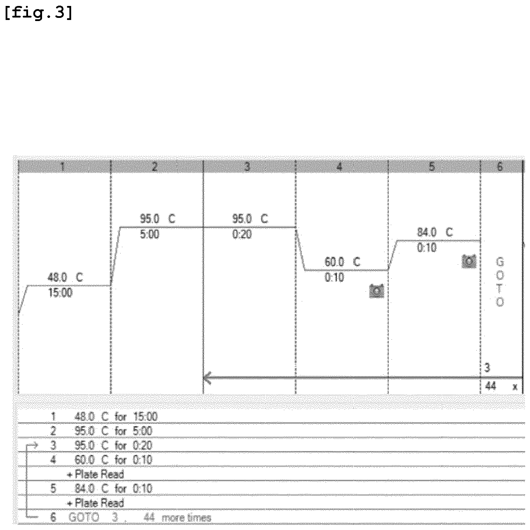

FIG. 3 is a schematic diagram of a method for measuring the fluorescence of the PCR reaction and the amplification product using the Bio-rad CFX 96 device;

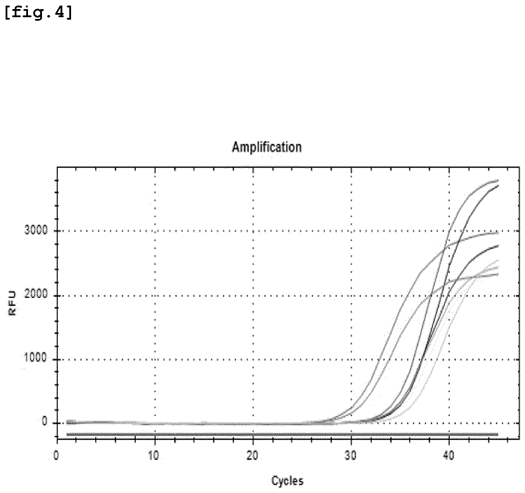

FIG. 4 is an optical curve measured at 60°C using a Bio-rad CFX96 device and is an amplification curve in which the amounts of the fluorescence signal derived from the amplification product of the internal control and the fluorescence signal derived from the amplification product of the target sequence are combined;

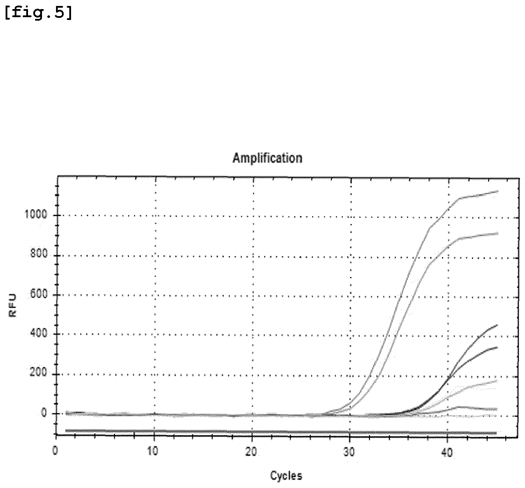

FIG. 5 is an optical curve measured at 84°C using a Bio-rad CFX96 device. The amplification product of the internal control is denatured at a temperature of 79°C or higher and thus does not exhibit fluorescence; FIG. 5 is an optical amplification curve in which a fluorescence signal derived from an amplification product of an internal control sequence is removed from the amplification curve of FIG. 4, and is an amplification curve is composed only of a fluorescence signal derived from an amplification product of a target sequence;

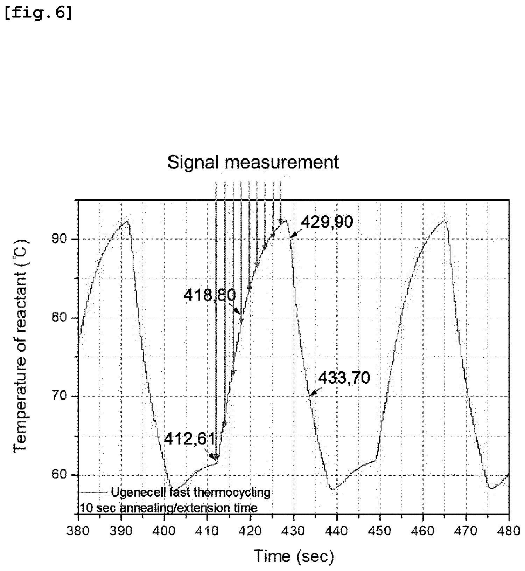

FIG. 6 is a schematic diagram showing a method of measuring the PCR reaction and fluorescence of an amplification product using a device to which the present disclosure is applied;

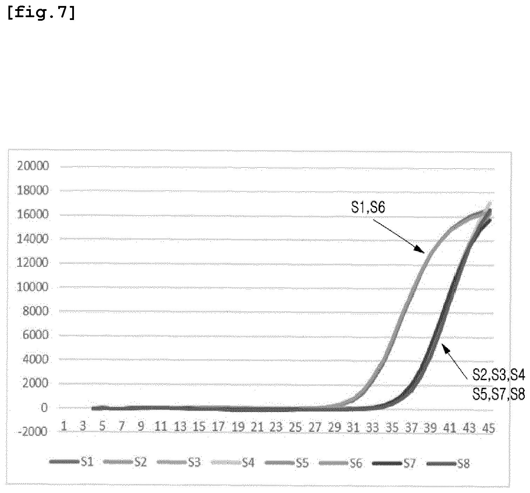

FIG. 7 is an optical curve is composed of data of an optical measurement sequence 1 using a device to which the present disclosure is applied and is an amplification curve that is the sum of the fluorescence signal derived from the amplification product of the internal control sequence and the amount of fluorescence derived from the amplification product of the target sequence; and

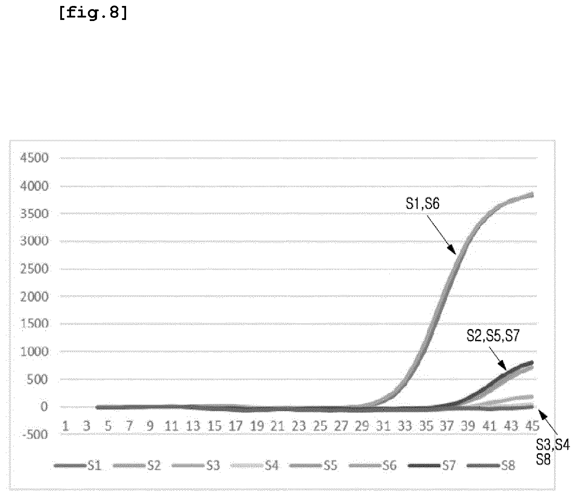

FIG. 8 is an optical curve is composed of data of sequence 11 optically measured using the device to which the present disclosure is applied and is an amplification curve of the signal excluding the fluorescence signal derived from the amplification product of the internal control sequence in the amplification curve of FIG. 7 and is an amplification curve is composed only of the fluorescence signal derived from the amplification product of the target sequence.

## Best Mode

## Mode for Disclosure

Hereinafter, the present disclosure will be described in detail through examples. The following examples only illustrate the present disclosure, but the scope of the present disclosure is not limited to the following examples.

[Example]

Example 1: Confirmation of target nucleic acid detection

1) HCV viral RNA detection

A control material of the Quanti-HCV v1.0 kit was extracted and mixed with a PCR reaction solution to detect HCV viral RNA. 20 $\mu$l of a PCR reaction solution containing different concentrations of control IVT RNA was added to each of the reaction vessel strips to which several PCR tubes were connected, and after closing the lid of the vessel, pre-denaturation for 1 minute in a heating block heated to 98°C was performed. The denaturation step was performed by contact with the heating block for 8 seconds, and an artificial air flow was provided by a blowing fan to cool for 9 seconds to perform annealing and DNA extension steps. PCR reaction was performed by repeating this process 45 times, and the specific PCR reaction solution, PCR device, and reaction conditions are shown in Table 1 below:

[Table 1]

| Condition | The present disclosure | Related art |
|---|---|---|
| RNA extraction | - Internal control gene and HCV RNA extraction: Viral RNA extraction kit (SEF-016, Ugenecell, KR) | |
| PCR reaction vessel | Bioplastic 0.1 mL tube strip(low profile) | |
| PCR amplification instrument | ExAmplar (Ugenecell, KR) | CFX 96 (Bio-rad, US) |
| PCR reaction solution | -PCR mixture: Primer (forward: 5'-GTTCTGCGGAACCGGTGAGTACA-3'/reverse: 5'-CGCRACCCAACRCTACTCGGCTA-3'; R is A or G) - Real MOD 2X RT-PCR enzyme mix (iNtRon, KR) | |
| PCR amplification conditions | A. Reverse transcription (48°C, 15 min) B. Initial denaturation (98°C, 5 min) C. Heating (98°C (contact heating block), 15 seconds) - Measure signals at 0.8 seconds intervals for 15 seconds of heating (represented by sequence) D. Cooling (9 seconds) E. Standing (12 seconds) C-E: Repeat 45 times | A. Reverse transcription (48°C, 15 min) B. Initial denaturation (98°C, 5 min) C. Heating (95°C, 20 seconds) D. Amplification, first optical measurement (60°C, 10 seconds) E. Secondary optical measurement (84°C, 10 seconds) C-E: Repeat 45 times |

13 IU and 300 IU of HCV target RNA were added to the reaction tube, respectively, and 10 IU of internal control RNA was added to all tubes. NCs without the addition of HCV target RNA (negative control) were also tested. The fluorescence of the amplification products was measured through the optics of each amplifier. In the above experiment, in order to compare with the conventional PCR method, a commercially available real time PCR device (CFX 96 of Bio-rad Corporation) was used. The PCR reaction using Bio-rad's CFX 96 was performed as shown in FIG. 3, the optical curve measured at 60°C is shown in FIG. 4, and the optical curve measured at 84°C is shown in FIG. 5.

The PCR reaction using the device of the present disclosure was performed as shown in FIG. 6, and the optical curves accordingly are shown in FIGS. 7 and 8, respectively, in which FIG. 7 is composed of the signal of sequence 1, and FIG. 8 is composed of the signal of sequence 11.

As a result of the experiment, as can be compared in FIGS. 4, 5, and 7, 8, in the amplification curve (FIG. 4) obtained through the general signal measurement method used in the conventional real time PCR device, since the signal by target sequence amplification and the signal by internal gene sequence amplification were overlapped, it was difficult to distinguish only the signal of the target sequence of interest. Therefore, in order to distinguish only the signal of the target sequence, the measurement temperature had to be adjusted to remove the signal by amplifying the internal gene sequence (FIG. 5). On the other hand, in the PCR device to which the present disclosure is applied, it was confirmed that it is possible to detect only the fluorescence signal of the amplification product for the target sequence because the fluorescence signal of the internal control group sequence may be excluded according to the measurement time point of the fluorescence signal (FIGS. 7 and 8).

**Claims**

1.  A method for detecting and quantifying a target nucleic acid in real time, the method comprising:

    (a) repeatedly performing DNA denaturation, annealing, and DNA extension of a target sequence and an internal control sequence, using a PCR reaction solution containing a single-signal fluorescent material and a primer pair that specifically bind to the target sequence under continuous temperature control; and
    (b) measuring a fluorescence signal at regular intervals from the start of heating for DNA denaturation to the completion of DNA denaturation.

2. The method of claim 1, wherein the single-signal fluorescent material in step (a) is an intercalating dye.

3. The method of claim 1, wherein the internal control sequence in step (a) is used to remove a false negative of the PCR reaction.

4. The method of claim 1, wherein the continuous temperature control in step (a) comprises heating a reaction vessel to a first temperature by bringing the reaction vessel containing the PCR reaction solution into contact with a heating block, and then cooling the vessel to a second temperature by separating the heated reaction vessel from the heating block and exposing the separated reaction vessel to an artificial air flow for a predetermined period of time, and the first temperature is a temperature at which the DNA denaturation is performed, and the second temperature is a temperature at which the annealing and/or the DNA extension is performed.

5. The method of claim 1, wherein the continuous temperature control in step (a) comprises:

   heating the reaction vessel to a first temperature by bringing the reaction vessel containing the PCR reaction solution into contact with the heating block;
   cooling the reaction vessel to a third temperature by separating the heated reaction vessel from the heating block and then exposing the separated vessel to an artificial air flow for a predetermined period of time; and
   heating the cooled reaction vessel to a second temperature by bringing the cooled reaction vessel into contact with the heating block and then separating the reaction vessel from the heating block,
   wherein the first temperature is a temperature at which the DNA denaturation is performed, the third temperature is a temperature at which the annealing is performed, and the second temperature is a temperature at which the DNA extension is performed.

6. The method according to claim 4 or 5, wherein, in the cooling of the reaction vessel, the heating block is fixed at a position, and the reaction vessel is moved upward to a predetermined position from the heating bock and so that the reaction vessel and the heating block are separated from each other.

7. The method of claim 6, wherein, in the cooling of the reaction vessel, the artificial air flow is continuously supplied.

8. The method according to claim 4 or 5, wherein, in the cooling of the reaction vessel, the reaction vessel is fixed at a position, and the heating block is moved downward to a predetermined position under the reaction vessel so that the reaction vessel and the heating block are separated from each other.

9. The method of claim 8, wherein, in the cooling of the reaction vessel, the artificial air flow is supplied only in a state in which the heating block is separated from the vessel.

10. The method according to claim 4 or 5, wherein, in the cooling of the reaction vessel, a predetermined time period is determined by the following general Formula 1.

[General Formula 1]

$$t = 4 + 2 * e^{-(v-7.4)/6.2}$$

In the above general Formula 1, t means a predetermined time, and v is the speed of the artificial air flow.

11. The method according to claim 4 or 5, wherein the reaction vessel and the heating block are spaced by a distance of 0.5 to 2 cm in a state in which the reaction vessel and the heating block are separated from each other.

12. The method of claim 4, wherein when only the annealing is performed at the second temperature, the method further comprises separating the reaction vessel from the heating block again after bringing the reaction vessel that is cooled to a second temperature into contact with the heating block so that the reaction vessel is heated to a fourth temperature, wherein the fourth temperature is a temperature at which the DNA extension is performed.

13. The method of claim 1, wherein, in step (b), the fluorescence signal is measured at regular time intervals from the start of the heating to the completion of the DNA denaturation,

the fluorescence signal measured at a time point Tx among the measured fluorescence signals is selected, and the fluorescence signal measured at the time point Tx is a fluorescence signal measured at a time point at which a temperature higher than the melting point of an amplified product of the internal control sequence is reached.

14. The method of claim 13, wherein, in step (b), the fluorescence signal (signal B) measured at the start of the heating comprises a fluorescence signal of an amplified product of the target sequence and a fluorescence signal of an amplified product of the internal control sequence, and
the fluorescence signal (signal A) measured at the time point Tx comprises only the fluorescence signal of the amplified product of the target sequence.

15. The method of claim 1, wherein the interval in step (b) is 0.5 to 1 second.

16. The method of claim 1, wherein the method further comprises checking whether there is a false negative by checking whether the internal control sequence is amplified or not.

[fig.1a]

[fig.1b]

[fig.2a]

Heating

Cooling

[fig.2b]

[fig.3]

| 1 | 2 | 3 | 4 | 5 | 6 |

95.0  C
5:00

95.0  C
0:20

84.0  C
0:10

48.0  C
15:00

60.0  C
0:10

G
O
T
O

3

44    x

1    48.0  C  for  15:00
2    95.0  C  for  5:00
3    95.0  C  for  0:20
4    60.0  C  for  0:10
     + Plate Read
5    84.0  C  for  0:10
     + Plate Read
6    GOTO   3 .    44   more times

[fig.4]

[fig.5]

[fig.6]

Signal measurement

[fig.7]

[fig.8]

## INTERNATIONAL SEARCH REPORT

| International application No. |
| --- |
| **PCT/KR2020/012411** |

**A.   CLASSIFICATION OF SUBJECT MATTER**

**C12Q 1/6851**(2018.01)i

According to International Patent Classification (IPC) or to both national classification and IPC

**B.   FIELDS SEARCHED**

Minimum documentation searched (classification system followed by classification symbols)

C12Q 1/6851(2018.01); B01L 7/00(2006.01); C12M 1/36(2006.01); C12N 15/09(2006.01); C12Q 1/68(2006.01); C12Q 3/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 타겟 핵산(target nucleic acid), 검출(detecting), 정량(quantifying), PCR, 변성 (denaturation), 형광 신호(fluorescent signal)

**C.   DOCUMENTS CONSIDERED TO BE RELEVANT**

| Category* | Citation of document, with indication, where appropriate, of the relevant passages | Relevant to claim No. |
| --- | --- | --- |
| Y | KR 10-2017-0080784 A (BIOCORE CO., LTD.) 11 July 2017 (2017-07-11) See abstract; claims 1-22; examples 1 and 2; and paragraphs [0018]-[0025]. | 1-13,15,16 |
| A | | 14 |
| Y | KR 10-2018-0090955 A (U-GENE&CELL CO. et al.) 14 August 2018 (2018-08-14) See abstract; claims 1-14; and examples 1 and 2. | 1-13,15,16 |
| A | AHBERG, C. D. et al. Single Fluorescence Channel-based Multiplex Detection of Avian Influenza Virus by Quantitative PCR with Intercalating Dye. Scientific Reports. 19 June 2015, vol. 5, document 11479, pp. 1-7. See entire document. | 1-16 |
| A | AHBERG, C. D. et al. Doubling Throughput of a Real-Time PCR. Scientific Reports. 27 July 2015, vol. 5, document 12595, pp. 1-9. See entire document. | 1-16 |

☑ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **16 December 2020** | **17 December 2020** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2020/012411** |

**C. DOCUMENTS CONSIDERED TO BE RELEVANT**

| Category* | Citation of document, with indication, where appropriate, of the relevant passages | Relevant to claim No. |
|---|---|---|
| A | US 7537886 B1 (NAZARENKO, I. et al.) 26 May 2009 (2009-05-26)<br>See entire document. | 1-16 |
| A | JP 2004-506431 A (UNIVERSITY OF UTAH RESEARCH FOUNDATION et al.) 04 March 2004 (2004-03-04)<br>See entire document. | 1-16 |
| A | US 2010-0075296 A1 (CLOAKE, M. et al.) 25 March 2010 (2010-03-25)<br>See entire document. | 1-16 |
| PX | KR 10-2107589 B1 (U-GENE&CELL CO. et al.) 07 May 2020 (2020-05-07)<br>See entire document. | 1-16 |

Form PCT/ISA/210 (second sheet) (July 2019)

**INTERNATIONAL SEARCH REPORT**

International application No.

**PCT/KR2020/012411**

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

## INTERNATIONAL SEARCH REPORT
### Information on patent family members

International application No.

**PCT/KR2020/012411**

| Patent document cited in search report | | | Publication date (day/month/year) | Patent family member(s) | | | Publication date (day/month/year) |
|---|---|---|---|---|---|---|---|
| KR | 10-2017-0080784 | A | 11 July 2017 | KR | 10-1855748 | B1 | 11 May 2018 |
| KR | 10-2018-0090955 | A | 14 August 2018 | CN | 110062659 | A | 26 July 2019 |
| | | | | KR | 10-2018-0087892 | A | 03 August 2018 |
| | | | | KR | 10-2039785 | B1 | 04 November 2019 |
| | | | | KR | 10-2081480 | B1 | 24 April 2020 |
| | | | | US | 2019-0329261 | A1 | 31 October 2019 |
| | | | | WO | 2018-139788 | A1 | 02 August 2018 |
| US | 7537886 | B1 | 26 May 2009 | AU | 2006-200604 | A1 | 16 March 2006 |
| | | | | AU | 5882000 | A | 09 January 2001 |
| | | | | CA | 2377707 | A1 | 28 December 2000 |
| | | | | EP | 1190097 | A2 | 27 March 2002 |
| | | | | JP | 2003-510017 | A | 18 March 2003 |
| | | | | NZ | 516278 | A | 26 March 2004 |
| | | | | NZ | 528967 | A | 24 March 2005 |
| | | | | US | 2011-0143350 | A1 | 16 June 2011 |
| | | | | US | 2016-0108466 | A1 | 21 April 2016 |
| | | | | WO | 00-79009 | A2 | 28 December 2000 |
| | | | | WO | 00-79009 | A3 | 17 January 2002 |
| | | | | WO | 00-79009 | A9 | 13 June 2002 |
| JP | 2004-506431 | A | 04 March 2004 | AT | 332398 | T | 15 July 2006 |
| | | | | CA | 2417986 | A1 | 21 February 2002 |
| | | | | CA | 2417986 | C | 26 November 2013 |
| | | | | DE | 60121342 | T2 | 28 June 2007 |
| | | | | EP | 1307592 | A2 | 07 May 2003 |
| | | | | EP | 1307592 | B1 | 05 July 2006 |
| | | | | ES | 2267803 | T3 | 16 March 2007 |
| | | | | JP | 5213297 | B2 | 19 June 2013 |
| | | | | US | 2003-0022177 | A1 | 30 January 2003 |
| | | | | US | 6635427 | B2 | 21 October 2003 |
| | | | | WO | 02-014555 | A3 | 27 February 2003 |
| | | | | WO | 02-14555 | A2 | 21 February 2002 |
| US | 2010-0075296 | A1 | 25 March 2010 | CA | 2638458 | A1 | 31 January 2010 |
| | | | | US | 8945880 | B2 | 03 February 2015 |
| KR | 10-2107589 | B1 | 07 May 2020 | | None | | |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020160126092